# EUROPÄISCHE PATENTSCHRIFT

(11) **EP 0 548 193 B1**
(45) Veröffentlichungstag und Bekanntmachung des Hinweises auf die Patenterteilung: **28.05.2003**
(21) Anmeldenummer: 91916472.3
(22) Anmeldetag: 13.09.1991
(51) Int. Cl.: A61L 24/00, A61L 27/34

(54) **MEHRKOMPONENTENMATERIAL UND VERFAHREN ZU SEINER HERSTELLUNG**
MULTIPLE COMPONENT MATERIAL AND PROCESS FOR PRODUCING THE SAME
MATERIAU A COMPOSANTS MULTIPLES ET SON PROCEDE DE PRODUCTION

(30) Priorität: 13.09.1990 DE 4029136; 19.09.1990 DE 4029714
(43) Veröffentlichungstag der Anmeldung: 30.06.1993
(60) Teilanmeldung: 01129733.0
(73) Patentinhaber: DRAENERT, Klaus, Dr.med., D-81545 München (DE)
(72) Erfinder: DRAENERT, Klaus, D-8000 München 90 (DE); WAHLIG, Helmut, D-6100 Darmstadt (DE)
(86) Internationale Anmeldenummer: EP9101746
(87) Internationale Veröffentlichungsnummer: WO92004924

(56) Entgegenhaltungen:
- EP-A- 0 041 614
- EP-A- 0 047 971
- DE-A- 2 905 878
- FR-A- 2 344 280

## Beschreibung

Die Erfindung betrifft ein Mehrkomponentenmaterial, insbesondere ein Zweikomponentenmaterial, mit Zusätzen, beispielsweise in Form von Füllerpartikeln. Inshesondere betrifft die Erfindung ein Implantationsmaterial, welches ein Polymer und/oder Copolymer sowie Füllerpartikel enthält. Derartige Implantationsmaterialien werden dazu verwendet, um Prothesenkomponenten im Knochen zu verankern und um den Knochen zu versteifen, sowie als Dübel von Knochenschrauben bzw. als Implantate zur Verankerung von Schrauben, beispielsweise bei Verbundosteosynthesen. Als Polymer werden dabei insbesondere polymere Acrylate, insbesondere Polymethylacrylate verwendet. Die Erfindung wird nachstehend anhand derartiger Implantationsmaterialien beschrieben, sie umfaßt jedoch jedes Mehrkomponentenmaterial, das aus mindestens einer polymeren Komponente und mindestens einer monomeren Komponente sowie Zusätzen, insbesondere partikelförmigen Zusätzen besteht.

Die vorstehenden Implantationsmaterialien sind als Knochenzemente bekannt und Klinisch erprobt. Sie werden vom Körper nicht resorbiert, sind jedoch biologisch inert und werden in das Knochengerüst integriert. Bekannte Knochenzemente bestehen aus einer polymeren Komponente, die in der Regel in Form von Kügelchen vorliegt und auch als Perlpolymerisat bezeichnet wird, einer monomeren Komponente und gegebenenfalls einem Polymerisationskatalysator, einem Stabilisator und einem Beschleuniger. Vor dem Gebrauch werden die Komponenten möglichst homogen vermischt. Knochenzemente auf Acrylatbasis haben sich als vorteilhafte Verankerungswerkstoffe für Prothesenkomponenten erwiesen. Der Nachteil derartiger Knochenzemente kann jedoch darin bestehen, daß trotz verbesserter Misch- und Applikationsmethoden die Materialfestigkeit und die Reproduzierbarkeit noch nicht verläßlich optimiert werden konnten. Die Knochenzemente können in ihrem klinischen Verlauf ermüden und zeigen eine zunehmende Zerrüttung, in deren Folge Knochenresorption und die dann nicht zu vermeidende Auslockerung des Implantates auftreten können.

Den genannten Implantationsmaterialien werden in der Regel Füllerpartikel zugesetzt, insbesondere um eine gewisse Porosität zu schaffen, die durch Nachwachsen von Knochengewebe zu einem besseren Verbund zwischen Implantat und Knochen führen soll.

Aus der DE-A-29 05 878 ist bekannt, daß eines der Probleme von Füllerpartikel aufweisenden Knochenzementen, sogenannten Füllerzementen, darin besteht, daß die Poren der Füllerpartikel Monomer aufsaugen und daß dieses Monomer dadurch nicht mehr für die Einbettung des Polymerpulvers zur Verfügung steht. Dies kann zu einer unvollständigen Polymerisation und zum Eindringen des Monomeren in den Kreislauf des Patienten führen. Außerdem können die Poren nicht in der gewünschten Weise ausgebildet werden, und es wird gegebenenfalls auch die Materialfestigkeit des Knochenzements herabgesetzt. In der DE-A-29 05 878 wird versucht, diese Probleme dadurch zu lösen, daß das Porenvolumen temporär verschlossen wird, um das Eindringen von Monomer zu verhindern. Allerdings sollen die Füllerpartikel dem Knochen möglichst frei zugänglich sein und können nicht völlig in den Knochenzement eingebettet sein, da sonst ihre freie Oberfläche dem Knochen nicht länger zugänglich wäre.

Neben den genannten Füllerpartikeln, die gamäß der DE-A-29 05 878 vorzugsweise aus resorbierbarem Tricalciumphosphat bestehen, können beispielsweise auch Röntgenkontrastalttel und Antibiotika als Füllerpartikel im allgemeinen Sinne verstanden werden. Der Begriff "Füllerpartikel" soll im Hahmen der Erfindung somit auch beispielsweise Röntgenkontrastmittel und Antibiotika einschließen. Es wurde bereits nachgewiesen (Lee A.J.C., Ling R.S.N., Vangala, S.S., Arch. Orthop. Traumat. Surg. 92, 1-18, 1978), daß auch Röntgenkontrastmittel und Antibiotika zu einer deutlichen Schwächung der Materialfestigkeit des Knochenzements führen, welche zwischen 4 % und 25 % liegen kann. Es wurde ebenfalls bereits nachgewiesen, (Klaus Draenert, Forschung und Fortbildung in der Chirurgie des Bewegungsapparats 2, zur Praxis der Zamentverankerung, Art und Science, München, Seite 39, 1988, ISDM 3-923112-11-4), daß insbesondere die Röntgenkontrastmittel Fülldefekte zur sekundär polymerisierenden Matrix des Knochenzements erkennen lassen.

In einer Normalpackung Knochenzement mit 40 g Polymerpulver sind bis zu 6 g Röntgenkontrastmittel, üblicherweise in Form von Bariumsulfat oder Zirkondioxid zugesetzt, wobei auch Wismutverbindungen verwendbar sind. Aus der DE-A-29 05 878 ist auch bekannt, daß Hydroxylapatit in entsprechender Beimengung einen ausreichenden Röntgenkontrast des Knochenzementimplantates ergeben kann. Die Röntgenkontrastmittel erfüllen ihre Funiction als röntgendichtes Material im Gegensatz zu den porösen Füllerpartikeln im engeren Sinne auch dann, wenn sie im Knochenzement eingebettet sind.

Neben der Schwächung der Materialfestigkeit des Knochenzements weist der Zusatz von Röntgenkontrastmittel der Füller noch das Problem auf, daß die Beimengung dieser Füller und vor allem die Reproduzierbarkeit der Homogenität des Gemisches nicht unerhebliche Probleme bei der industriellen Herstellung des Polymerpulvers verursachen kann. Es hat sich gezeigt, das es trotz ausgefeilter Technik mit Kugelmühle und verschiedenen Rüttlern immer wieder vorkommt, daß einzelne Packungen Knochenzement sogenannte Konglomerate von Röntgenkontrastmittel beinhalten, die zu einem Aufsaugen von Monomer führen mit der Folge, daß sich die Anrührbedingungen und die Viskosität dieser Knochenzsmentcharge von der der üblichen Knochenzemente gänzlich unterscheiden können. Auch wurde festgestellt, daß sich die Röntgenkontrastmittel, beispielsweise das Zirkondioxid, verschiedener Hersteller unterscheiden, und daß insbesondere Bariusasulfat zu den genannten Konglomeratbildungen neigt.

Die deutsche Offenlegungsschrift DE-A-27 27 535 beschreibt u.a. Antibiotikaadditive für Knochenzement. Die EP-A-47 971 betrifft Dentalmaterialien aus polymerisierbaren Massen, die kunststoffbeschichtete mineralische Füllerpartikel aus Alumosilikaten bzw. Gläsern mit einer Größe von 0,1 - 20 µ enthalten. Das Patent US-A-4 500 658 offenbart Röntgenkontrastmittel enthaltenden Knochenzement. Der Knochenzement wird hergestellt, indem ein Acrylat in Gegenwart des Kontrastmittels auspolymerisiert und dann vermahlen wird.

Der Erfindung liegt somit die Aufgabe zugrunde, die vorstehenden Nachteile zu vermeiden und ein Zusätze aufweisendes Mehrkomponentenmaterial, insbesondere Zweikomponentenmaterial bereitzustellen, bei dem die partikelförmigen Zusätze keine Schwächung der Materialfestigkeit des Mehrkomponentenmaterials und keine Fülldefekte hervorrufen.

Insbesondere liegt der Erfindung die Aufgabe zugrunde, die vorstehenden Nachteile zu vermeiden und ein Füllerpartikel aufweisendes Implantationsmaterial, wie Knochenzement bereitzustellen, bei dem die Füllerpartikel keine Schwächung der Materialfestigkeit des Implantationsmaterials und keine Fülldefekte hervorrufen. Eine weitere Aufgabe der Erfindung besteht darin, die Füllerpartikel homogen und reproduzierbar im Implantationsmaterial zu verteilen. Ferner liegt der Erfindung die Aufgabe zugrunde, ein Verfahren zur Herstellung von Zusätze, insbesondere partikelförmige Zusätze enthaltendem Mehrkemponentenmaterial, insbesondere von Füllerpartikel enthaltendem Implantationsmaterial mit den genannten vorteilen bereitzustellen.

Diese Aufgabe wird durch das Mehrkomponentenmaterial, insbesondere das Implantationsmaterial, und das Verfahren gemäß der Erfindung gelöst. Die Erfindung geht dabei von dem Grundgedanken aus, geeignete Maßnahmen zu treffen, daß die Polymerkomponente des Materials, die dafür sorgt, daß das selbsthärtende Gemisch aus Monomer und Polymer innerhalb der nützlichen und gewünschten zeitlichen Fristen aushärten kann, die Zusätze, z.B. die Füllerpartikel, in idealer Weise aufnelmen kann. Hierzu verden die Füllerpartikel oder andere Zusätze während der Herstellung des Materials von den Polymerpartikeln zumindest teilweise umschlossen, mit den Polymerpartikeln beschichtet oder auf andere Weise in diese eingebettet. Dadurch lassen sich die genannten Probleme beim Einbringen der Füllerpartikel oder anderer Zusätze in einfacher und eleganter Weise lösen.

In der vorliegenden Beschreibung wird der Begriff Polymer so verstanden, daß dabei auch Copolymere umfaßt werden. Vorzugsweise werden erfindungsgemäß Polymere auf der Basis eines Polyacrylats oder Polymethacrylats, Copolymere eines Acrylats und eines Methacrylats, Mischungen der genannten Polymere und Copolymere, Epoxidharz oder ein Polymer eines als Implantationsmaterial verwendbaren anderen Kunststoffes verwendet.

Die Füllerpartikel, beispielsweise Röntgenkontrastmittel, weisen vorzugsweise eine Größe von 5 bis 15 µm auf, die Polymerkugeln der Pulverkomponente sind dagegen größer und weisen beispielsweise für den herkömmlichen Palacos R®-Zement eine Größenverteilung zwischen 1 und 140 µm mit einem Maximum der Größen zwischen 60 und 80 µm auf. Die Partikel sind in der Ragel etwa kugelförmig und die vorstehenden Größenangaben beziehen sich in diesem Fall auf die Durchmesser der Kugeln. Falls die Partikel nicht kugelförmig sind, ist unter den vorstehenden Größenangaben der mittlere Durchmesser der Partikel zu verstehen. Die Füllerpartikel können eine Größe bis zu 250 µm und die Polymerpartikel eine Größe bis zu 300 µm aufweisen.

Bei dem erfindungsgemäßen Verfahren werden die kleineren Füllerpartikel, während der Herstellung der Polymerpartikel als Kristallisationskeim eingebettet, und es können auf diese Weise die gesamten für eine Normalpackung Knochenzement erforderlichen 6 g Röntgenkontrastmittel in die Polymerkomponente aufgenommen werden, ohne daß zusätzliche Füllerpartikel frei dem Polymerpulver zugefügt werden müssen. Auf diese Weise kann vermieden werden, daß eine zusätzliche Porosität entsteht, die zur Aufnahme von Monomerflüssigkeit geeignet wäre. Außerdem ist sichergestellt, daß das Röntgenkontrastmittel in dem weitaus stabileren Polymer der Pulverkomponente und nicht in der weniger widerstandsfähigen Matrix des zugesetzten Monomers bei dessen Auspolymerisierung eingebaut wird.

Durch die Einbettung der Partikel z.B. des Röntgenkontrastmittels in das polymer wird das Röntgenkontrastmittel in seiner Funktion als röntgendichtes Material nicht beeinträchtigt. Als Röntgankontrastmittel werden vorzugsweise CrO₂, BaSO₄, Bi₂O₃, Bi(OH)₃ und/oder flüssige oder feste jodhaltige Kontrastmittel verwendet.

Die Füllerpartikel können auch aktive Wirkstoffe enthalten. Es hat sich nämlich gezeigt, daß aktive Wirkstoffe, beispielsweise Antibiotika, auf dem Diffusionswege freigesetzt werden, auch wenn sie zunächst in die Polymerpartikel eingebettet oder von diesen beschichtet sind. Deshalb können erfindungsgemäß auch derartige Wirkstoffe, beispielsweise Antibiotika, zumindest zu ihrem größten Teil in die Polymerpulverkomponente aufgenommen werden. Als Antibiotika werden vorzugsweise Gentamycin und/oder Clindamycin verwendet.

Auch andere Wirkstoffe, die sukzessive auf dem Diffusionswege aus dem Polymer freigesetzt werden, sind als Füllkörper in das Polymer einbettungsfähig.

Als einbettungsfähige Füllerpartikel können beispielsweise auch Hydroxylapatit, Tricalciumphosphat oder eine andere Calciumphosphat- oder Calciumcarbonatverbindung, in die Polymerpartikel eingebettet oder mit diesen beschichtet werden. Es sind auch Kombinationen aller genannten Füllerpartikel möglich.

Gemäß der DE-A-29 05 878 beträgt das Porenvolumen der Tricalciumphosphat-Füllerpartikel vorzugsweise weniger als 0,1 ml/g. Bei dem erfindungsgemäßen Implantationsmaterial können die Füllerpartikel eine hohe Porosität aufweisen, da sie in das Polymer eingebettet oder von diesem beschichtet sind. Vorzugsweise können die Füllerpartikel eine Porosität von 20 bis 80 % aufweisen. Es können auch Füllerpartikel mit einer Porosität von mindestens 50 % verwendet werden. Andererseits ist es auch möglich, dichte Füllerpartikel mit einem Porenvolumen von nahezu 0, die lediglich eine Mikroporosität aufweisen, in das erfindungsgemäße Implantationsmaterial einzubringen.

Zur Herstellung des erfindungsgemäßen Implantationsmaterials gemäß Anspruch 1 sind alle Verfahran geeignet, mit denen sich die Füllerpartikel zumindest teilweise mit dem Polymer beschichten oder in dieses einbetten lassen. Hierfür sind auch verschiedene an sich bekannte und geläufige Verfahren geeignet. Beispielsweise kann die Beschiehtung dadurch erfolgen, daß das Polymer zusammen mit den Füllerpartikeln im Sprühverfahren in einer Fällungslösung ausgeformt wird. Die Beschichtung oder Einbettung der Füllerpartikel in das Polymer kann auch durch Ausfällung, durch elektrostatische Beschichtung, durch Beschichtung im Tauchverfahren oder durch Beschichtung im Pelletierverfahren oder andere bekannte Verfahren erfolgen.

Der Einschluß von Röntgenkontrastmittelpartikeln, aber auch der anderen, bereits genannten Füllerpartikel, kann in besonders einfacher und bevorzugter Weise dadurch erreicht werden, daß ein herkömmlicher Knochenzement, vorzugsweise in Form von Perlpolymerisat, mit homogen eingemischten Füllern, beispielsweise Zirkondioxid-Röntgenkontrastmittelpartikeln, geschmolzen wird und danach unter hohem Druck über eine geeignete Düse in ein Fällungsbad ausgepreßt wird, beispielsweise mittels einer Spritzgußmaschine. Dabei werden in der Regel Drücke von etwa 300 bar angewandt, der Druck kann aber auch bis 2000 bar betragen. Je kleiner die ausgefällten Teilchen sein sollen, umso feiner auß die Düse sein, und um so höher muß der angewandte Druck sein. Bei herkömmlichen Knochenzementen, wie z.B. Palacos R®-Zement, wird bei einer Temperatur von etwa 190 bis 255°C gearbeitet, da das Polymer bei Temperaturen etwas über 250°C verbrennt. Die Füller mit einer Größe von 5 bis 15 µm bieten mit ihrer unregelmäßigen Oberfläche der Polymermatrix eine große Haftfläche, was zu einem sicheren Einschluß der Füller in die Polymerkomponente führt. Auch im Fällungsbad bleibt das Polymer am längsten an den Füllerpartikeln hängen, so daß alle Füllerpartikel inkorporiert bleiben. Dieses Herstellungsverfahren ist preisgünstig und bietet darüber hinaus auch den Vorteil, daß Ausgangsmaterialien, die bereits klinisch erprobt sind, für die Herstellung des Implantationsmaterials verwendet werden können.

Es ist auch möglich, das Polymermaterial als kompakten Block beliebiger Form herzustellen und diesen dann zu einem Granulat in geeigneter Teilchengröße zu zerkleinern. Dabei entstehen keine Polymerkugeln, sondern unregelmäßig geformte Teilchen. Es hat sich gezeigt, daß beim Einsatz einer so gewonnenen Polymerkomponente Knochenzemente mit deutlich verbessertan mechanischen Eigenschaften erhalten werden. Es ist dafür nicht notwendig, daß die gesamte Polymerkomponente aus einem solchen Granulat besteht; es sollten jedoch zumindest 1 bis 50 Gew.-% sein, wobei mit 5 bis 20 Gew.-% schon sehr gute Resultate erzielt werden.

Wenn die Füllerpartikel in diesen Polymerblock eingebracht werden, z.B. durch Einarbeiten in die Schmelze oder durch Einmischen in die Monomerflüssigkeit und anschließende Polymerisation, werden beim Zermahlen sowohl Granulatteilchen mit eingeschlossenen Füllerpartikeln als auch ohne solche erhalten. Es ist möglich, ein solches Granulat direkt einzusetzen. Alternativ kann auch ein Granulat ohne Füllerpartikel zusammen mit auf andere Weise gewonnenen polymerumhüllten Füllerpartikeln eingesetzt werden.

Der Einsatz eines aus einem Polyerblock durch Zerkleinern gewonnenen unregelmäßigen Granulats ist auch unabhängig von der Verwendung der polymerumhüllten Fülldrpartikel der vorliegenden Anmeldung sehr vorteilhaft. Auch bei üblichen Knochenzeaenten, in denen Füllerpartikel frei vorliegen, wird durch Ersatz des kugelförmigen Präpolymerisats durch zumindest 1 bis 50 Gew.-% eines Granulats eine verbesserte mechanische Stabilität erreicht.

Die erfindungsgemäßen Implantationsmaterialien lassen sich in hervorragender Weise zur Verankerung von Prothesenkomponenten im Knochen und zur Versteifung von Knochen verwenden. Das erfindungsgemäße Implantationsmaterial kann ferner auch als Dübel von Knochenschrauben bzw. als Implantat zur Verankerung von Schrauben, beispielsweise bei der Verbundosteosynthese, verwendet werden.

Ein Vorteil des erfindungsgemäßen Implantationsmaterials liegt darin, daß keine negative Beeinflussung des Anmischverhaltens bei der Verwendung des Implantationsmaterials beispielsweise als Knochenzement auftreten kann, womit die Reproduzierbarkeit gleichartiger Chargen erheblich verbessert werden kann. Ein weiterer Vorteil liegt darin, daß eine wesentlich gleichmäßigere Wirkstoff-Freisetzung aus dem Implantationsmaterial in wesentlich höheren Konzentrationen erfolgen kann. Letztlich liegt ein Vorteil auch darin, daß der geringere Monomerverbrauch die Temperaturentwicklung während der Aushärtung des Implantationsmaterials verringert, die mechanische Stabilität des Materials drastisch erhöht, und der Röntgenkontrast sehr viel gleichmäßiger verteilt wird.

### Beispiel 1

1000 g eines Polymethylmethacrylat-Granulates werden zusammen mit 150 g als Röntgenkontrastmittel dienendem Zirkondioxid geschmolzen, und die Schmelze wird anschließend über eine Düse in ein Fällungsbad ausgepreßt. Dabei werden Polymerkugeln erzeugt, in die das Zirkondioxid eingebettet ist. Die erzeugten Polymerkugeln werden anschließend über verschiedene Siebe gesiebt und getrocknet und schließlich in einer Auswahl zwischen 1 und 140 µm gesammelt, abgefüllt, mit Benzylperoxid versetzt und fertig abgepackt.

### Beispiel 2

1000 g eines Polymethylmethacrylat-Granulates werden durch Hitze verflüssigt und getrennt mit 150 g Zirkondioxid in einer Zeratäubungsanlage in ein Fällungsbad ausgepreßt. Dabei werden die Zirkondioxidpartikel mit Polymethylmethacrylat beschichtet, und die das Zirkondioxid enthaltanden Polymethylmethacrylat-Kugeln sammeln sich in dem Fällungsbad und werden in der in Beispiel 1 beschriebenen Weise ausgesiebt und unter Zusatz von Benzylperoxid abgepackt.

### Beispiel 3

1000 g eines herkömmlichen Polymethylmethacrylat-Perlpolymerisates werden zusammen mit 150 g als Röntgenkontrastmittel dienendem Zirkondioxid homogen durchmischt und danach in einer Kammer auf 250°C erhitzt und dadurch verflüssigt. Die Schmelze wird anschließend mit Drücken von etwa 300 bar durch eine Düse in ein Fällungsbad ausgespritzt. Dabei werden Folymerkugeln erzeugt, in die das Zirkondioxid eingebettet ist. Die erzeugten Polymerkugeln werden anschließend über verschiedene Siebe gesiebt und getrocknet und schließlich in einer Auswahl zwischen 1 und 140 µm gesammelt, abgefüllt, mit Benzylperoxid versetzt und fertig abgepackt.

## Patentansprüche

1. Implantationsmaterial bestehend aus einem Polymer und/oder Copolymer und einem Monomeranteil, gegebenenfalls aus einem Stabilisator, Katalysator und Beschleuniger, so beschaffen, dass das Polymer und/oder Copolymer in Form von Polymerteilchen vorliegt, die
(i) einen oder mehrere Wirkstoffe ganz oder teilweise umschliessen, und/oder
(ii) einen oder mehrere Füllerpartikel auf der Basis von Hydroxylapatit, Tricalciumphopshat oder einer anderen Calciumphosphat-oder Calciumcarbonatverbindung einer Teilchengrösse bis 250 µm ganz oder teilweise umschliessen.

2. Implantationsmaterial nach Anspruch 1, wobei die Füllerpartikel in das Polymer und/oder Copolymermaterial vor dessen Auspolymerisierung eingebettet sind.

3. Implantatmaterial nach Anspruch 1 oder 2, wobei das Implantationsmaterial ein Polymer auf der Basis eines Polyacrylates, Polymetacrylates, Polymethylacrylates, Polymethylmetacrylates, ein Copolymer eines Acrylates und eines Metacrylates, ein Epoxidharz und/oder ein Poymer eines als Implantationsmaterial verwendbaren Kunststoffes enthält.

4. Implantationsmaterial nach einem der Ansprüche 1 bis 3, wobei die Partikel des Polymerund/oder Copolymermaterials, welches die Füllerpartikel zumindest teilweise umschliessen, eine Grösse vorzugsweise bis 300 µm aufweisen.

5. Implantationsmaterial nach einem der Ansprüche 1 bis 4, wobei als Wirkstoff Antibiotika zugesetzt sind.

6. Implantationsmaterial nach einem der Ansprüche 1 bis 5, wobei als Antibiotikum Gentamycin und/oder Clindamycin zugesetzt sind.

7. Implantationsmaterial nach einem der Ansprüche 1 bis 6, wobei die Füllerpartikel eine Porosität von 20 bis 80% haben.

8. Luylautationsnaaterial nach einem der Ansprüche 1 bis 7, wobei die Füllerpartikel dicht sind und ihr Porenvolumen annähernd 0 ist.

9. Implantationsmatcrial nach einem der Ansprüche 1 bis 8, wobei die Polymer-und /oder Copolymerteilchen Kugelform haben.

10. Implantationsmaterial nach einem der Ansprüche 1 bis 8, wobei die Polymer-und /oder Copolymerteilchen Granulatform haben.

11. Implantationsmaterial nach einem der Ansprüche 1 bis 10, wobei ein Gemisch aus Granulat mit teilweisem oder ganzem Füllereinschluss und anderen Polymer-und/oder Copolymerteilchen verwandt wird.

12. Implantationsmaterial nach einem der Ansprüche 1 bis 11, wobei der Granulatantcil 1 - 50 Gew.-% ausmacht.

13. Implantationsmaterial nach einem der Ansprüche 1 bis 12, wobei diese zusätzlich Röntgenkontrastmittel wie ZiO2, BaSO4, Bi2O3, Bi(OH)3 und/oder flüssige oder feste jodhaltige Kontrastmittel als Füllerpartikel enthalten.

14. Verfahren zur Herstellung eines Implantationsmsterials nach einem der Ansprüche 1 bis 13, wobei die Füllerpartikel mit dem Polymer-und/oder Copolymermaterial beschichtet werden.

15. Verfahren zur Herstellung eines Implantationsmaterisls nach einem der Ansprüche 1 bis 13, wobei die Füllerpartikel in das Polymer-und/oder Copolymermaterial eingebettet werden.

16. Verfahren zur Herstellung eines Implantatmaterials nach einem der Ansprüche 14 oder 15, wobei die Beschichtung oder Einbettung durch ein Sprühverfahren oder durch Ausfällung erfolgt.

17. Verfahren zur Herstellung eines Implantatiansmaterisls nach einem der Ansprüche 14 oder 15, wobei die Beschichtung oder Einbettung durch elektrostatische Beschichtung, Beschichtung im Tauchverfahren oder Beschichtung im Pelletierverfahren erfolgt.

18. Verfahren zur Herstellung eines Implantationsmaterials nach einem der Ansprüche 1 bis 13, wobei das Poylmer und/oder Copolymermaterial zusammen mit den Füllerpartikeln im Sprühverfahren in einer Pällungslösung ausgeformt wird.

19. Verfahren zur Herstellung eines Implantationsmaterials nach einem der Ansprüche 1 bis 13, wobei die Füllerpartikel in einer Schmelze von dem Perlpolymerpulver des Polymers und/oder Copolymers ausgenommen werden und die Schmelze durch eine Düse in ein Fällungsbad ausgespritzt wird.

20. Implantationsmaterial bestehend aus einem Polymer und/oder Copolymermaterial und einem Monomeranteil, gegebenenfalls aus einem Stabilisator, Katalysator und Beschleuniger, so beschaffen, dass das Polymer und/oder Copolymer in Form von Poylmerteilchen vorliegen, die Füllerpartikel als Röntgenkontrastmittel einer Teilchengrösse bis 250µm ganz oder teilweise umschliessen, so herstellbar, dass die Füllerpartikel in einer Schmelze von dem Perlpolymerpulver des Polymers und/oder Copolymers und/oder Granulat des Polymeren aufgenommen werden und die Schmelze durch eine Düse in ein Fällungsbad ausgespritzt wird.

21. Auspolymerisierbares Implantationsmaterial nach Anspruch 20 dadurch ausgezeichnet, dass als Röntgenkontrastmittel ZrO2, BaSO4, Bi2O3, Bi(OH)3 und /oder jodhaltige Kontrastmittel verwandt werden.

## Claims

1. An implantation material comprising a polymer and/or a copolymer and a monomer portion, if possibly of a stabiliser, catalyst and accelerator, formed in such a way that the polymer and/or copolymer is present in the form of polymer particles which
(i) completely or partially enclose one or several active ingredients, and/or
(ii) completely or partially enclose one or several filler particles on the basis of hydroxyapatite, tricalcium phosphate or another calcium phosphate or calcium carbonate compound with a particle size of up to 250 µm.

2. An implantation material according to Claim 1, wherein the filler particles are embedded in the polymer and/or copolymer material before they are polymerised out.

3. An implantation material according to Claim 1 or 2 wherein the implantation material contains a polymer on the basis of a polyacrylate, polymetacrylate, polymethylacrylate, polymethylmetacrylate, a copolymer of an acrylate and a metacrylate, an epoxy resin and/or a polymer of a plastic which can be used as an implantation material.

4. An implantation material according to any of Claims 1 to 3, wherein the particles of the polymer and/or copolymer material which is at least partially enclosed by the filler particles, preferably exhibit a size of up to 300 µm.

5. An implantation material according to any of Claims 1 to 4, wherein antibiotics are added as an active ingredient.

6. An implantation material according to any of Claims 1 to 5, wherein gentamycin and/or clindamycin are added as antibiotic.

7. An implantation material according to any of Claims 1 to 6, wherein the filler particles have a porosity of between 20 and 80%.

8. An implantation material according to any of Claims 1 to 7, wherein the filler particles are dense and their pore volume is almost 0.

9. An implantation material according to any of Claims 1 to 8, wherein the polymer and/or copolymer particles are of spherical form.

10. An implantation material according to any of Claims 1 to 8, wherein the polymer and/or copolymer particles are in granular form.

11. An implantation material according to any of Claims 1 to 10, wherein a mixture of granulate with partial or total filler inclusion and other polymer and/or copolymer particles is used.

12. An implantation material according to any of Claims 1 to 11, wherein the granulate portion amounts to 1 -50% by weight.

13. An implantation material according to any of Claims 1 to 12, wherein these additionally contain X-ray contrast material such as ZrO2, BaSO4, Bi2O3, Bi(OH)3 and/or liquid or solid contrast material containing iodine as filler particles.

14. A process for the manufacture of an implantation material according to any of Claims 1 to 13, wherein the filler particles are coated with the polymer and/or copolymer material.

15. A process for the manufacture of an implantation material according to any of Claims 1 to 13, wherein the filler particles are embedded in the polymer and/or copolymer material.

16. A process for the manufacture of an implantation material according to Claim 14 or 15 where the coating or embedding is implemented by means of a spray process or by means of precipitation.

17. A process for the manufacture of an implantation material according to Claim 14 or 15 wherein the coating or embedding is implemented by means of electrostatic coating, coating by means of a dipping process or coating using the Pelletier process.

18. A process for the manufacture of an implantation material according to any of Claims 1 to 13, wherein the polymer and/or copolymer material is formed together with the filler particles in a spray process in a precipitation solution.

19. A process for the manufacture of an implantation material according to any of Claims 1 to 13, wherein the filler particles are received in a melted mass of the pearl polymer powder of the polymer and/or copolymer and the melted mass is sprayed through a nozzle into a precipitation bath.

20. An implantation material consisting of a polymer and/or copolymer material and a monomer portion, possibly a stabiliser, catalyst and accelerator, formed in such a way that the polymer and/or copolymer is present in the form of polymer particles, which enclose filler particles in the form of X-ray contrast material of a particle size of up to 250 µm completely or partially, which can be manufactured in such a way that the filler particles are taken up into a melted mass of the pearl polymer powder of the polymer and/or copolymer and/or granulate of the polymers and the melted mass is sprayed out into a precipitation bath through a nozzle.

21. An implantation material which can be polymerised out according to Claim 20 **characterised in that** ZrO2, BaSO4, Bi2O3, Bi(OH)3 and/or contrast material containing iodine are used as X-ray contrast material.

## Revendications

1. Matériau pour implants constitué d'un polymère et/ou d'un copolymère et en partie d'un monomère, le cas échéant d'un stabilisateur, d'un catalyseur et d'un accélérateur, composé de telle sorte que le polymère et/ou le copolymère se présente(nt) sous forme de fragments de polymère (i) englobant totalement ou partiellement plusieurs substances, et/ou (ii) englobant totalement ou partiellement une ou plusieurs particules de charge à base d'une hydroxyle-apatite, de phosphate tricalcique ou d'un autre phosphate de calcium ou d'une combinaison de carbonate de calcium dont la grosseur des particules atteint jusqu'à 250 µm.

2. Matériau pour implants selon la revendication 1, les particules de charge étant logées dans le polymère et/ou dans le copolymère avant sa dépolymérisation.

3. Matériau pour implants selon la revendication 1 ou 2, le matériau pour implants renfermant un polymère sur la base d'un polyacrylate, d'un polyméthyle-acrylate, d'un polyméthyle-métacrylate, d'un copolymère d'acrylate ou de métacrylate, d'une résine époxy et/ou d'un polymère de matière plastique utilisable comme matériau pour implants.

4. Matériau pour implants selon les revendications 1 à 3, les particules du polymère et/ou du matériau de copolymère qui englobent au moins partiellement les particules de charge présentant de préférence une grosseur allant jusqu'à 300 µm.

5. Matériau pour implants selon l'une des revendications 1 à 4, le principe actif ajouté étant des antibiotiques.

6. Matériau pour implants selon l'une des revendications 1 à 5, les antibiotiques ajoutés étant de la gentamycine et/ou de la clindamycine.

7. Matériau pour implants selon l'une des revendications 1 à 6, la porosité des particules de charge étant de 20 à 80 %.

8. Matériau pour implants selon l'une des revendications 1 à 7, les particules de charge étant denses et le volume de leurs pores approchant 0.

9. Matériau pour implants selon les revendications 1 à 8, les fragments de polymère et/ou de copolymère ayant une forme sphérique.

10. Matériau pour implants selon l'une des revendications 1 à 8, les fragments de polymère et/ou de copolymère ayant la forme de granulés.

11. Matériau pour implants selon l'une des revendications 1 à 10, le mélange utilisé étant constitué par des granulés renfermant une inclusion partielle ou totale de charge et d'autres fragments de polymère et/ou de copolymère.

12. Matériau pour implants selon l'une des revendications 1 à 11, la part de granulés constituant une part pondérale de 1 à 50 %.

13. Matériau pour implants selon l'une des revendications 1 à 12, comportant en supplément en tant que particules de charge des produits de contraste pour radiographie tels que ZrO2, BaSO4, Bi203, Bi(OH)3 et/ou des produits de contraste liquides ou solides renfermant de l'iode.

14. Procédé de fabrication d'un matériau pour implants selon l'une des revendications 1 à 13, les particules de charge étant enrobées dans une couche de polymère et/ou de copolymère.

15. Procédé de fabrication d'un matériau pour implants selon l'une des revendications 1 à 13, les particules de charge étant incluses dans le polymère et/ou le copolymère.

16. Procédé de fabrication d'un matériau selon l'une des revendications 14 ou 15, l'enrobage ou l'inclusion étant réalisé par précipitation.

17. Procédé de fabrication d'un matériau selon l'une des revendications 14 ou 15, l'enrobage ou l'inclusion étant réalisé par dépôt électrostatique, revêtement par immersion ou selon le procédé Pelletier.

18. Procédé de fabrication d'un matériau pour implants selon l'une des revendications 1 à 13, le polymère et le copolymère étant configurés ensemble avec les particules de charge et par pulvérisation dans une solution donnant un précipité.

19. Procédé de fabrication d'un matériau pour implants selon l'une des revendications 1 à 13, les particules de charge étant retirées dans une coulée de la poudre de perles du polymère et/ou du copolymère et la coulée étant injectée à travers une buse dans un bain de précipitation.

20. Matériau pour implants constitué d'un polymère et/ou d'un copolymère et en partie d'un monomère, le cas échéant d'un stabilisateur, d'un catalyseur et d'un accélérateur, composé de telle sorte que le polymère et/ou le copolymère se présente(nt) sous forme de fragments de polymère, les particules de charge étant un produit de contraste pour radiographie dont la grosseur des fragments atteint jusqu'à 250 µm, englobant totalement ou partiellement, pouvant être fabriqué de telle sorte que les particules de charge soient intégrées dans une coulée de la poudre de perles du polymère et/ou du copolymère et/ou des granulés du polymère, la coulée étant injectée à travers une buse dans un bain de précipitation.

21. Matériau pour implants dépolymérisable selon la revendication 20, **caractérisé par le fait que** l'on utilise comme produit de contraste pour radiographie ZrO2, BaSO4, Bi203, Bi(OH)3 et/ou des produits de contraste renfermant de l'iode.
